# EUROPEAN PATENT SPECIFICATION

(11) **EP 3 629 020 B1**
(45) Date of publication and mention of the grant of the patent: **27.10.2021**
(21) Application number: 18306247.0
(22) Date of filing: 25.09.2018
(51) Int. Cl.: G01N 33/50, B01L 3/00, G01N 33/543, G01N 33/58

(54) **MICROFLUIDIC DROPLET-BASED ASSAY PROCESS AND APPARATUS**
VERFAHREN UND VORRICHTUNG FÜR AUF MIKROFLUIDISCHEN TRÖPFCHEN BASIERENDEN TEST
PROCÉDÉ ET APPAREIL DE DOSAGE DE GOUTTELETTES MICROFLUIDIQUES

(43) Date of publication of application: 01.04.2020
(73) Proprietor: bioMérieux, 69280 Marcy-l'Etoile (FR); Bioaster, 69007 Lyon (FR)
(72) Inventor: VEDRINE, Christophe, 78420 Carrières-sur-Seine (FR); BOUNAB, Yacine, 92600 Asnière sur Seine (FR)
(74) Representative: Cabinet Beau de Loménie

(56) References cited:
- WO-A1-2013/041983
- US-A1- 2010 279 374
- US-A1- 2017 205 404
- SUNGJUN YOON ET AL: "Droplet-based microfluidic system to form and separate multicellular spheroids using magnetic nanoparticles", LAB ON A CHIP, vol. 13, no. 8, 1 January 2013 (2013-01-01), page 1522, XP55527000, ISSN: 1473-0197, DOI: 10.1039/c3lc41322e
- KLAUS EYER ET AL: "Single-cell deep phenotyping of IgG-secreting cells for high-resolution immune monitoring", NATURE BIOTECHNOLOGY, vol. 35, no. 10, 11 September 2017 (2017-09-11), pages 977-982, XP55504042, ISSN: 1087-0156, DOI: 10.1038/nbt.3964

## Description

The invention lies within the field of microfluidic droplet-based assay processes and of apparatus for analyzing biological cell activity by detection of at least one target analyte contained in one or several droplets.

One application of the invention relates to the characterization of cell secretion dynamics, i.e. the evolution of such secretion over time.

Studies have shown that cell secretion dynamics and their heterogeneity play a fundamental role in physiological and pathological processes. The characterization of heterogeneous dynamic responses at the single cell level is crucial for understanding biological systems and to design effective therapeutic interventions. For instance, dynamic profiling of cancer cell secretome has permitted the discovery of novel biomarkers for cancer diagnostics and to evaluate therapies. To protect the organism against invaders, the immune system mobilizes a set of dynamic processes that includes signaling molecule secretions, cell differentiation, cell migration as well as phagocytosis. Immune responses of individual cells to external cues such as, for instance, simple exposure to a homogeneous ligand, are highly dynamic and display dramatic cell-to-cell variations. One key feature in immune reaction is the production and secretion of a large panel of signaling cytokines that coordinate the immune functions. Deciphering cytokine dynamic processes is necessary to understand mechanisms underlying immunity and to help building predictive models for the immune system and future therapies. However, owing to limitations of current methodologies, little is known about cytokine dynamic profiles in the context of individual cells. The most commonly used technologies to study production and secretion of cytokines at single cell resolution are flow cytometry and Enzyme-linked immunospot (ELISPOT) assay, respectively. However, these single-time-point-based technologies do not enable full understanding of cell functions dynamics. In addition, these approaches use large volumes of both samples and assay reagents. The introduction of miniaturization of laboratory procedures has held great potential for single cell-based assays (see Hummer, D., Kurth, F., Naredi-Rainer, N. & Dittrich, P. S. "Single cells in confined volumes: microchambers and microdroplets" - Lab chip, 2016, 447-458, referred to hereinafter as *"Hummer et al., 2016"*)*.* In particular, novel technologies, based on microfabrication and microfluidics, have been developed to assess cytokine secretion from single cells. Micro-engraving technology uses a dense array of polydimethylsiloxane (PDMS)-based nanowells for simultaneous dynamic measurements of multiple cytokines that are secreted by individual cells. It can be noted that this technology suffers from complicated device fabrication processes and, in some cases, complex handling of cells and assay reagents. In addition, non-specific absorption of secreted proteins by PDMS-based nanowells might affect the sensitivity and reproducibility of the assay. Also, single-cell barcode chip (SCBC) technology for multiplexed analysis and droplet microfluidics assays for high-throughput screening have already been developed for cytokine secretion. However, as previously mentioned, these endpoint approaches do not assess the dynamic nature of cytokine-secreting cells.

Thus, there remains the need for a simple and robust droplet-based microfluidic assay which, when combined with optical imaging, including for example, time-lapse fluorescence microscopy, enables assessing the functional dynamics of individual cells, for example of immune cells.

Single-cell biology assays can benefit considerably from the unique capabilities offered by droplet microfluidics systems (see Linas Mazutis, John Gilbert, W Lloyd Ung, David A Weitz, Andrew D Griffiths, John A Heyman - « Single-cell analysis and sorting using droplet-based microfluidics »- Nature Protocols, 8(5), 870-891 (2013), hereinafter referred to as *"Mazutis et al., 2013"*). The cell and bioassay reagents are confined into highly monodisperse droplets with volumes ranging from pico- to nanoliters. Each droplet serves as an independent bioreactor to assess biochemical cellular reactions, thus enabling ultrahigh-throughput screening and analysis of large numbers of single cells. Droplet microfluidics combined with time-lapse microscopy is suitable for fast and quantitative real-time detection of the molecule of interest secreted by the single cell (*Mazutis et al*., 2013; *and* Klaus Eyer, Raphaël C L Doineau, Carlos E Castrillon, Luis Briseño-Roa, Vera Menrath, Guillaume Mottet, Patrick England, Alexei Godina, Elodie Brient-Litzler, Clément Nizak, Allan Jensen, Andrew D Griffiths, Jérôme Bibette, Pierre Bruhns & Jean Baudry: "Single-cell deep phenotyping of IgG-secreting cells for high-resolution immune monitoring" ; published in NATURE BIOTECHNOLOGY, volume 35, number 10, October 2017*,* hereafter referred to as *"Eyer et al., 2017"*). The compartmentalization of single cells together with ultrasensitive bioassays into picoliter volumes accelerates detection of biological responses. Indeed, upon activation, the molecules secreted by individual cells rapidly accumulate within droplets and rapidly reach detectable concentrations; thus only a few molecules are needed to reach the limit of detection. For example, 3000 molecules are sufficient to reach a 0.1nM limit of detection (LOD) in 50-pL droplet, which would be equivalent to 6x10⁹ molecules in the 100 µL of a microtiter-plate-based assay.

Another advantage of droplet-based assays is the possibility to fine-control the environment of the confined single cell and thus to assess the functional dynamics and diversity of cells in well-defined conditions. Moreover, microfluidic-based cell assays are an attractive analytical tool for working with low volume specimens obtained from biohazardous origins or with precious clinical samples (*Mazutis et al., 2013*).

Examples of microfluidic droplet-based assay processes and apparatus are described in a document by:
- Jöensson, H., & Svahn, H. A. (2012) - "Droplet Microfluidics-A Tool for SingleCell Analysis"; published in Angewandte Chemie International Edition, 51(49), 12176-12192; DOI: 10.1002/anie.201200460*;* hereafter referred to as *"Jöensson et al., 2012"*
   and in a document by
- Yu Fen Samantha Seah, Hongxing Hu, Christoph A. Merten; "Microfluidic single-cell technology in immunology and antibody screening"; published in Molecular Aspects of Medicine 59 (2018), 47-61; available at *https:*//*doi.org*/*10.1016*/*j.mam.2017.09.004;* hereafter referred to as *"Seah et al., 2018"*

As an example of analyzing biological cell activity, here in relation to immune cell secretion dynamic, the inventors have devised a process and an apparatus to assess functional dynamic of primary human monocyte at single cell resolution. Monocytes are key cells of the innate immune system for inflammatory responses. Human monocytes represent 10% of circulating leukocytes and are well known to exhibit heterogeneity in term of morphology, surface markers, endocytosis activity as well as signaling molecules secretome.

The exemplary bioassay which will be detailed hereinafter is based on single monocyte compartmentalization in 50-pL droplets together with nanobeads binding assay to reveal cytokine secretion and with fluorescent probes to assess cell viability. Lipopolysaccharide (LPS) is also confined within the droplet to activate the monocyte and trigger the secretion of cytokines. Following the joint encapsulation of a cell and bioassay reagents, the resulting emulsion is transferred into a glass storage chamber. The chamber content is imaged with an automated fluorescence microscope at regular time interval, with an image resolution enabling analysis of single droplet content.

The inventors have successfully activated and monitored the time course behavior of a large number of single CD14 human monocytes. Cells remained alive within the droplets for over the course of the experiment and the ultrasensitive homogenous fluorescent-based binding assay enabled fast and rapid detection of TNF-alpha secretion dynamics at the single cell level. A 30 min stimulation at 37 °C was sufficient to detect secreted TNF-alpha from a single monocyte. Thus, the sensitivity of the process and apparatus according to the invention exceeds that of conventional single cell secretion assays such as ELISPOT. Unlike endpoint measurement assays, the inventor's approach provided quantitative measurement of TNF-alpha secretion rate, allowing a deep characterization of monocyte functional dynamic profiles. This multidimensional analysis revealed a complex picture of TNF-alpha secretion dynamics of single CD14 monocyte.

The process and apparatus could be used to assess other types of biological activity, including by analyzing secretion dynamics of signaling molecules and cell heterogeneity of a diverse range of both immune and non-immune cells in biological samples. This may have clinical diagnostic relevance for diseases associated with cell dynamic response and their heterogeneity.

In essence, the invention deals with a microfluidic droplet-based assay process for analyzing biological cell activity by detection of at least one target analyte contained in one or several droplets, and deals with an apparatus for implementing such a process. The process comprises the step of creating one or several assay droplets of a fluid, typically an aqueous liquid, more typically a liquid culture medium where an assay droplet contains:
- paramagnetic particles;
- detectable markers;
- and, at least one biological cell potentially capable of releasing at least one target analyte.

The process relies on the paramagnetic particles and the detectable markers being able to bind with a target analyte to form a marked and magnetized combination with a target analyte. Then, several assay droplets are stored along a single layer, along a horizontal storage plane.

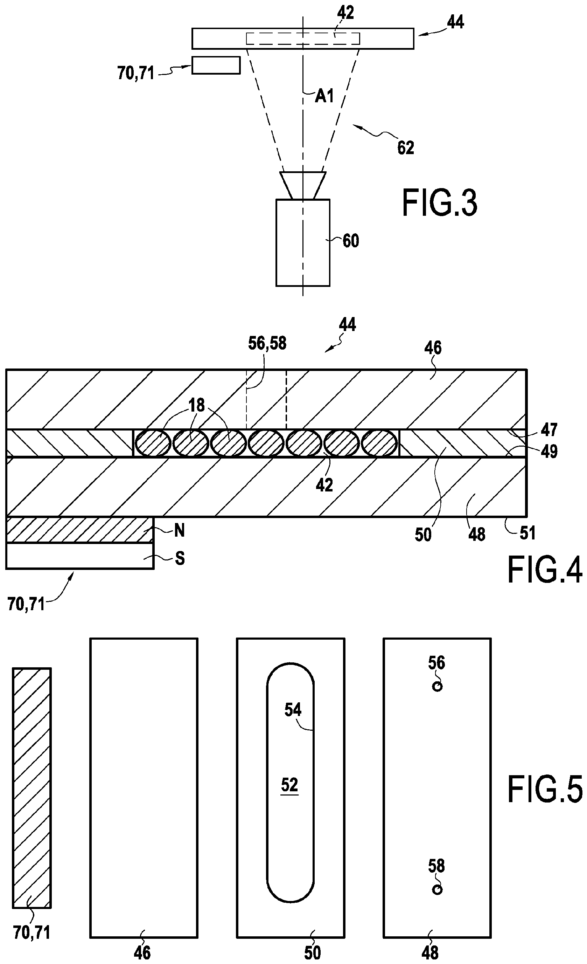

Such a process and apparatus are described in *Eyer et al., 2017.* This document describes such a process and apparatus which include the generation of a magnetic field through assay droplets stored in storage chamber for spatially arranging the paramagnetic particles inside the stored assay droplet. The process and apparatus use a detection method based on optical imaging. Optical imaging is performed along an imaging axis **A1** (see **Fig. 3****),** which is here substantially perpendicular to the horizontal storage plan, i.e. vertically. Spatially arranging the paramagnetic particles inside the droplets may include grouping the marked and magnetized combinations together geographically in the droplets, which may make the detectable markers more visible, due to a local higher concentration at the location where the marked and magnetized combinations are grouped together in the droplets.

Document WO 2016/059182 discloses a similar process and apparatus. With such a process and apparatus, wherein the magnetic field is generated by an assembly of two magnets which are located on two opposites sides of the storage chamber, the secretion of the target analyte is highlighted by the observation of a relocation and concentration of the fluorescence to form an elongated object, more or less fluorescent, extending in the droplet from one side to the other.

Although being very promising, the process and apparatus described in these two documents allow only a detection and/or a measurement of a florescence that is supposed to reflect the possible release of a target analyte. These described process and apparatus provide no information about the cells encapsulated in the monitored droplets, neither their number (possibly their absence) nor their physiological state, thenceforth the generated results are binary and very basic, which must necessarily come from the observation of a large number of droplets. A positive signal (*i.e.* a positive detection of a fluorescence a relocation and concentration of the fluorescence inside the droplet) recorded for a significant number of droplets is supposed to be associated with a positive detection of the targeted activity. A negative signal recorded with almost all the droplets is conversely associated with a non-response of the cell for the targeted activity.

Additionally, in the event that an exploitation of these processes and apparatus is wished in an optical imaging field, more particularly in cell imaging field, the magnetic field which is generated, here by an assembly of two magnets which are located on two opposite sides of the storage chamber, leads to some difficulties in obtaining proper and usable images. Indeed, in the process and the method, it has been found that it was difficult to obtain images in which both the cell and the detectable markers could be pictured with enough precision and image quality to be able to measure, in the image, a proper physical characteristic of the detectable marker, at the level of an individual droplet. In order to obtain usable images at the level of an individual droplet, it is indeed necessary to avoid having, in a given droplet, the grouped marked and magnetized combination and the cell aligned along the optical imaging axis **A1.** Indeed, in such a case, one would at least partially hide the other, which could lead to improper detection of the required physical property. Also, it has been experienced that proper focus was difficult to achieve, across one individual droplet and also between different cells in a given storage chamber. If any of the grouped marked and magnetized combinations and/or of the cell is out-of-focus in the image, the detection might be altered, especially when it involves evaluation or measurement of a physical characteristic in the image. This phenomenon can be at least in part attributed to the very shallow depth of field of the imaging devices, which are based on optical microscopes, used for imaging the droplets.

Thus, one aim of the invention is to provide an improved process and apparatus for analyzing biological cell activity which can be exploited in cell imaging field, where the optical images obtained for the detection step have a better focusing accuracy, allowing an assessment of both a physical property of the detectable marker and of the states of the cell(s) responsible for this recorded signal. One other aim of the invention is to provide an improved process and apparatus where the acquired optical images allow a follow-up in time of one or several individual encapsulated biological cells, with respect to their physiological state and at least a targeted secretion activity.

Another aim of the invention is to provide such an improved process and apparatus that could generate images that could be easily processed by an automated image processing software, and with which the relevant droplets for the study could be automatically identified, particularly thanks to the ascertainment that the individual encapsulated cells and the grouped marked and magnetized combinations are rightly located at their respective attributed locations inside the droplets.

The invention thus provides for a microfluidic droplet-based assay process for analyzing biological cell activity by detection of at least one target analyte contained in one or several droplets. The process comprises the step of creating several assay droplets of a liquid culture medium where an assay droplet contains:
- paramagnetic particles;
- detectable markers;
- and, at least one biological cell potentially capable of releasing at least one target analyte;
wherein the paramagnetic particles and the detectable markers are able to bind with a target analyte to form a marked and magnetized combination with a target analyte.

The process includes storing several assay droplets along a single layer along a horizontal storage plane.

The process includes the generation of a magnetic field through the stored assay droplets for spatially arranging the paramagnetic particles inside the stored assay droplets.

The process includes detecting, within a given stored assay droplet, a physical characteristic of the detectable markers contained in the given assay droplet, by optical imaging of the detectable markers.

The process is characterized in that the generation of a magnetic field generates a magnetic field such that the paramagnetic particles are attracted towards a lateral and inferior portion of the given stored assay droplet in which they are contained.

According to optional features of the process, taken alone or in combination :
- The magnetic field may be such that the paramagnetic particles are attracted towards a similarly disposed lateral and inferior portion of all the stored assay droplets.
- The magnetic field may be asymmetric with respect to the stored assay droplets.
- The process may comprise detecting fluorescence of fluorescent detectable markers and/or detecting radioisotope-labeled detectable markers.
- The process may comprise providing, in the assay droplet, additional detectable markers able to bind with specific proteins expressed on the biological cell surface, to tag the biological cell.
- The liquid culture medium may comprise at least one vital dye and/or at least one non-vital dye.

The invention also provides for an apparatus for a microfluidic droplet-based assay process for analyzing biological cell activity by detection of at least one target analyte contained in one or several droplets. Such apparatus comprises a storage chamber for storing several assay droplets of a liquid culture medium containing:
- paramagnetic particles;
- detectable markers;
- and, at least one biological cell potentially capable of releasing at least one target analyte.

In such apparatus, the storage chamber is transparent in a detection process of the detectable markers and where the storage chamber is formed between two parallel top and bottom inner wall surfaces of the storage chamber, the inner wall surfaces extending parallel to a horizontal plane and being separated vertically by a height, such that the assay droplets are stored along a single layer.

The apparatus includes a magnetic field generator for spatially arranging the paramagnetic particles inside the assay droplets stored in the storage chamber.

The apparatus includes a detector of a physical characteristic of the detectable markers contained in a given assay droplet stored in the storage chamber.

The apparatus is characterized in that the magnetic field generator generates a magnetic field such that the paramagnetic particles are attracted horizontally towards at least one side of the storage chamber and vertically towards the bottom inner wall surface of the storage chamber.

According to optional features of the apparatus, taken alone or in combination :
- The magnetic field generator may comprise one or several magnets forming a magnet assembly which is arranged below the level of the bottom inner wall surface of the storage chamber.
- The inner bottom wall surface of the storage chamber may pertain to a bottom wall of the storage chamber which exhibits a bottom external wall surface opposite its inner wall surface, and magnet assembly may be arranged below the level of the bottom external wall surface.
- The magnetic field generator may generate a magnetic field such that the paramagnetic particles are attracted horizontally towards a same side of the storage chamber.
- The magnetic field generator may comprise one or several magnets forming a magnet assembly and having each magnetic poles, wherein at least one of the poles of each of the magnets is located horizontally to a same side of the storage chamber, which may be the side towards which the paramagnetic particles are attracted horizontally.
- The magnetic field generator comprises one or several magnets forming a magnet assembly and having each magnetic poles, wherein both of the poles are located horizontally to a same side of the storage chamber, which may be the side towards which the paramagnetic particles are attracted horizontally.
- The storage chamber may be elongated along a first direction of the horizontal plane, and in that both of the poles are located horizontally to a same side along a second direction of the storage chamber which is perpendicular to the first direction, where the same side may be the side towards which the paramagnetic particles are attracted horizontally.
- The magnetic field generator may generate a magnetic field such that the paramagnetic particles are attracted, along the second direction, horizontally towards a side of the storage chamber.
- The side of the storage chamber may be defined with respect to a centroid of the extension of the storage chamber along the horizontal plane.
- The side of the storage chamber may be defined with respect to a periphery of the storage chamber along the horizontal plane.
- The storage chamber may be part of a sample carrier device and the magnetic field generator may be affixed to said sample carrier device.
- The magnetic field generator may be part of a detection station of the apparatus and the storage chamber may be part of a sample carrier device, the detection station and the sample carrier device being movable relative one to the other and being brought into coincidence for detection of at least one target analyte contained in one or several droplets contained in the storage chamber of the sample carrier device.

The invention also provides for a sample carrier which may comprise two plates forming the two walls of the storage chamber, in which the two plates may be affixed one to the other by an adhesive film sandwiched between the two plates, and wherein the thickness of the adhesive film determines vertically the height of the storage chamber. The adhesive film may comprise an internal cutout, the border of which delimits the extension of the storage chamber along the horizontal plane.

The processes and apparatus according to the invention will be further described in detail below with reference to the accompanying drawings showing some embodiments of the invention.

In the figures:
- **Figure 1** represents schematically a process for producing assay droplets which can be performed according to known technologies.
- **Figure 2** represents an example of how a paramagnetic particle, a target analyte and a detectable marker can bind to form a marked and magnetized combination.
- **Figure 3** represents schematically a detection station in an apparatus according to one possible embodiment of the invention.
- **Figure 4** represents schematically a vertical transverse cross section of a storage chamber with a magnetic assembly according to one possible embodiment of the invention.
- **Figure 5** represents a top view of the 4 main components of an embodiment of a sample carrier device comprising a storage chamber, before assembly.
- **Figure 6** represents schematically a top view of sample carrier device according to **Fig. 5****.**
- **Figures 7A, 7B** represent schematically a top view of some elements in a droplet, in the absence of a target analyte, at two different stages of a process, and **Figure 7C** represents a bright-field image of the droplet of **Fig. 7B** while **Figure 7D** is a schematic representation of the droplet of **Fig. 7B****,** visualized under a wavelength appropriate to excite the detectable markers of the droplet.
- **Figures 8A, 8B** represent schematically a top view of some elements in a droplet, in the presence of a target analyte, at two different stages of a process, and **Figure 8C** represents a bright-field photography of the same droplet as in **Fig. 8B** while **Figure 8D** is a schematic representation of the droplet of Fig.8B, visualized under a wave length appropriate to excite the detectable markers of the droplet.
- **Figures 9A** and **9B** represent a top view and cross section of a variant of the positioning of a magnet assembly with respect to a storage chamber, according to another embodiment of the invention.
- **Figures 10A** and **10B, 11A** and **11B, 12A** and **12B****,** are similar view representing further embodiments of the invention.
- **Figure 13** is an image of several droplets in a storage chamber, that can be obtained when the applied magnetic field is oriented as in the case of a magnetic assembly arranged as in **Fig. 4** and **Figs. 10A** and **10B, 11A** and **11B.**
- **Figure 14** is an image of several droplets in a storage chamber, that can be obtained when the applied magnetic field is oriented as in the case of a magnetic assembly arranged as in **Figs. 9A** and **9B, 12A and 12B****.**

The invention provides for a microfluidic droplet-based assay process for analyzing biological cell activity by detection of at least one target analyte contained in one or several droplets. It also provides for an apparatus for implementing such a process.

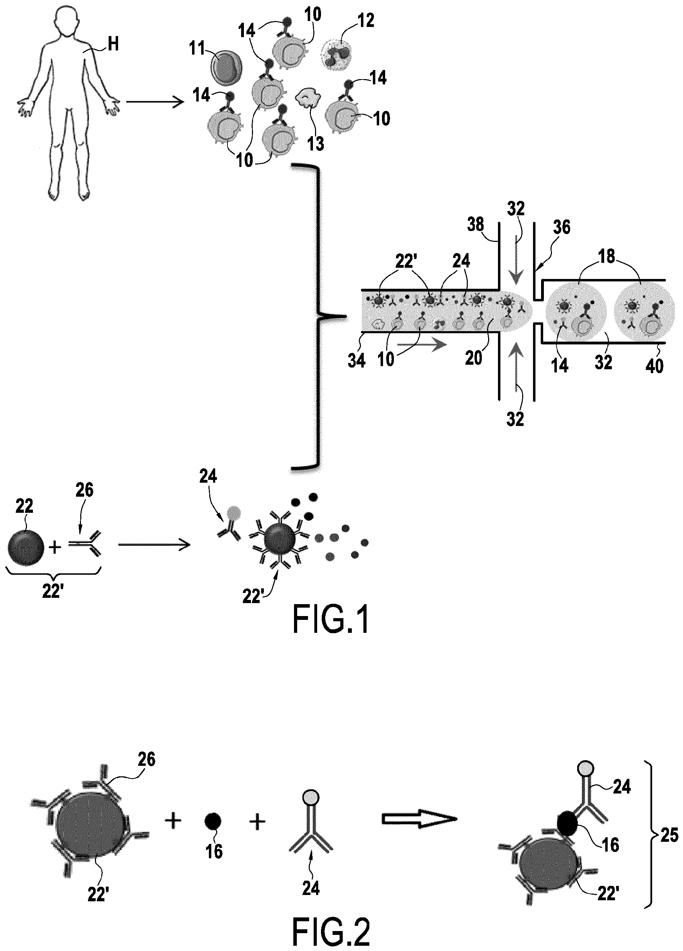

The apparatus and the process may be used for analyzing the activity of any type of biological cell, including eukaryotic cells (animal, plant, fungal and protozoan cells) or prokaryotic cells. In **Fig. 1** is represented schematically the extraction and preparation of biological cells **10, 11, 12,** for example from a living human **H.** The extraction may include live cells **10,** and may include dead cells **13.** In the exemplary application which will be referred hereafter, the biological cells comprised human monocytes **10** which were enriched using an isolation kit (for example as provided by StemCell™ Technologies, 40 Rue des Berges, Miniparc Polytec, Bâtiment Sirocco, 38000 Grenoble, France). Human monocytes can be specifically tagged by anti-CD14 fluorescent antibodies **14** to distinguish them from other cell-types **11, 12.**

It is here contemplated that, under certain circumstances, such cells might have a biological activity whereby they are able to produce, directly or indirectly, at least one certain species of analyte **16** (shown in **Fig. 2**), which will be detectable by a detection process.

In the process and apparatus according to the invention, it will be sought to detect such analyte which will hereinafter be called a target analyte. It will be possible to devise a process and an apparatus according to the invention where several analytes, by the meaning of different species of analytes, may be targeted, thus forming different target analytes. Such different target analytes could be the result, direct or indirect, of the same biological process inside the biological cell **10,** or of different biological processes inside the same biological cell **10.**

A target analyte **16** can be for example any protein that may be released by a biological cell, such as cytokines (e.g. chemokines, interferons, interleukins, lymphokines, tumour necrosis factors, growth factors), antibodies, lectins, hemoglobin, hormones, toxins. A target analyte **16** may also microRNAs (miRNAs), which have recently been disclosed as being possible circulating chemical mediators that would be involved in a particular form of intercellular communication. Therefore, such secreted miRNAs may also be contemplated as target analytes within the meaning of the invention. In the exemplary application, human monocytes **10** were studied for their aptitude to secrete TNF-alpha **16,** after stimulation by lipopolysaccharide (LPS) used as an active agent.

As schematically shown in **Fig. 1****,** the process comprises the steps of creating several assay droplets **18** of a suspension fluid, typically an aqueous liquid **20,** more typically a liquid culture medium **20,** where an assay droplet contains
- paramagnetic particles **22, 22';**
- detectable markers **24;**
- and, as discussed above, at least one biological cell **10** potentially capable of releasing at least one target analyte.

In the exemplary application, said suspension fluid was a cell culture medium ensuring cell viability throughout the course of the experiment. By "culture medium", it is meant a composition comprising nutritive elements necessary for the expression of a metabolism and/or the growth of the studied biological cell. Said culture medium is liquid, more particularly an aqueous liquid culture medium adapted to cell survival, at least during the assay. In addition to water, an aqueous culture medium generally comprises one or several of:
- a source of carbon and of energy (generally glucose, but according to the nature of the biological cell, saccharides such as lactose, maltose, etc... may be appropriate)
- a source of calcium (for example CaCl₂);
- a source of nitrogen and sulfur (for example (NH₄)₂SO₄);
- a source of magnesium (for example MgCl₂);
- trace elements (for example, salts of Cu, Zn, Co, Ni, B, and/or Ti).

Optionally, a culture medium may also comprise a pH buffer and/or various salts to maintain the medium at an appropriate pH and/or an appropriate osmolality.

Also, as will be detailed below, the suspension fluid may comprise, for example as part of the culture medium, at least one vital dye and/or at least one non-vital dye.

Additionally, the suspension fluid may contain, for example as part of the culture medium, at least one active agent potentially capable of interfering (stimulating, increasing, or inhibiting) with the release of said at least one target analyte **16** by the biological cell. The active agent may be a known activator/stimulator used to amplify and/or accelerate the secretion of the target analyte, with a view to improving the sensitivity of the assay and/or obtaining more accurate results and/or obtaining results more quickly. The active agent can also be a molecule that is tested (for example a drug candidate) for its ability to block the secretion of the target analyte or on the contrary to induce and/or enhance the secretion of this target analyte.

As shown in **Fig. 2****,** the paramagnetic particles **22, 22'** and the detectable markers **24** contained in an assay droplet **18** are able to bind with a target analyte **16** to form a marked and magnetized combination **25** with a target analyte **16.**

The paramagnetic particles **22** are attracted by an externally applied magnetic field. On the other hand, the paramagnetic particles **22** do not retain any magnetization in the absence of an externally applied magnetic field. Paramagnetic particles **22** may comprise paramagnetic beads. The paramagnetic particles **22** may comprise nanoparticles, which may typically exhibit a largest dimension comprised between 10 and 1000 nanometers, preferably between 100 and 500 nanometers. Typically, the paramagnetic particles **22** can be spherical beads, with a diameter of about 300 nanometers, i.e. comprised between 200 and 400 nanometers.

The paramagnetic particles **22** are preferably monodisperse, by exhibiting, within a given population, a maximum variation of the largest dimension of the particles which is less than preferably 20%, more preferably less than 10%, of the largest dimension of all paramagnetic particles in the population. Paramagnetic nanoparticles are well suited to immunodiagnostic technologies owing to their large surface-to-volume ratio, their monodispersity, their stability and easy manipulation in a magnetic field. They provide an important toolbox for chemical modification and functionalization of their outer surface. Moreover, some evidence showed that nanoparticle-based biosensors exhibit better homogeneity and sensitivity than microbead-based biosensors where the beads have a dimension larger than 1000 nanometers. The paramagnetic particles **22** may exhibit carboxyl (COOH) or amine (NH₂) groups on their outer surface, for facilitating the coupling of capture elements.

In the exemplary application, the used paramagnetic particles **22** were Carboxyl-Adembeads 0213 from Ademtech SA - Bioparc BioGalien - 27, allée Charles Darwin - 33600 PESSAC - France, as described in their protocol brochure 0213 v2.6.

In order to be able to bind specifically with a target analyte **16,** the paramagnetic particles **22** need to be functionalized. Functionalization of the paramagnetic particles comprises coupling, to said outer surface of the paramagnetic particles, capture elements **26** which are capable of binding with the target analytes **16.** Such capture elements **26** may be antibodies directed against target analytes **16,** for example G type immunoglobulin (IgG), monoclonal or polyclonal, directed against target analytes **16.** In the exemplary application, capture elements **26** were monoclonal anti-TNF-alpha IgG (ref. 3510-5-250, MABTECH, Sweden).

In the last decade, several antibody coupling procedures have been developed which can be implemented for coupling antibodies to the paramagnetic particles. In contrast to random coupling, it is preferred to implement procedures enabling an oriented coupling of the antibodies, such as IgG 26, onto the paramagnetic particles, so that the antigen-binding (Fab) fragment recognition sites of the antibody remain exposed. This improves the analyte detection and biosensor sensitivity. It is for example possible to use a boronic acid (BA) based coupling procedure for precise orientated coupling of IgGs on the surface of paramagnetic nanoparticles **22** (Duval F., van Beek T. A., Zuilhof, H. "Key steps towards the oriented immobilization of antibodies using boronic acids"; published in The Analyst, 140, 6467-6472 (2015)). BA molecules may be thus coupled on the surface of carboxylated beads to act as an affinity head-group for covalent and site-specific IgG attachment. The BA-based procedure maintains the integrity of IgG structure and immunoactivity. BA forms a cyclic boronate diester with the diols of glycan chains located on IgG crystallizable fragment Fc domain 30, far away from the active binding site. The procedure does not require modification of IgGs, thus preserving the biological activity of antibodies. There exists a large range of commercially available BA from different suppliers. Water-soluble BA molecules, reference A71751 from Merck (formerly, Sigma-Aldrich), a 3-aminophenylboronic acid hemisulfate salt, CAS Number 66472-86-4, have proven to provide optimal results.

Alternatively to antibodies, capture elements **26** may consist in entities known as natural or synthetic/mimetic of receptors or of ligands of said target analytes **16.**

Detectable markers **24** are chosen for their ability to combine with the target analyte **16.**

A detectable marker **24** may be an antibody, monoclonal or polyclonal, directed against target analytes **16,** and may have at least one physical characteristic which may be detected in a detection process. In this regard, an antibody used as a detectable marker in the sense of the invention is advantageously labelled with fluorescent dye or a radioisotope.

A detectable marker in the sense of the invention may be a fluorescent-labelled marker and/or a radioisotope-labelled marker that is able to bind with a target analyte.

In the exemplary application, the detectable markers **24** were second monoclonal anti-TNF-alpha IgG. Those second monoclonal anti-TNF-alpha IgG were different from the first monoclonal anti-TNF-alpha IgG used as capture elements **26** in that they bind to another part of the target analyte **16.** Additionally, they were coupled to phycoerythrin (PE), a fluorescent dye. Typically, in the exemplary application, the detectable markers **24** were PE-anti-TNF-alpha IgG (ref. 120-014-229, MILTENYI BIOTEC, Germany).

Alternatively to antibodies, detectable markers **24** may consist in entities known as natural or synthetic/mimetic receptors said target analytes**16** or of ligands of, possibly labelled with a fluorescent dye or a radioisotope.

Obviously, capture element **26** and detectable marker **24** are chosen such that they can bind simultaneously to a same target analyte **16.**

In the event that the detection of several target analytes is desired, then different detectable markers **24** labelled with different labels (such as different fluorescent dyes or radioisotopes) may be used; each of these detectable markers with its specific fluorescent dye or radioisotope being associated to a specific target analyte.

In addition to the detectable markers which are able to bind with the target analyte, the process may comprise providing, in the assay droplet, additional detectable markers which able to bind with the biological cell **10.** Such cell-directed additional detectable markers, which may also be fluorescent or radioisotope-labeled, are advantageously used in order to identify the cell-type, and confirm that the encapsulated cell fits with the cells to study. For example, detectable markers directed against CD14 and/or CD33 allow the identification of macrophages and monocytes, whereas detectable markers directed against CD3 and/or CD4 and/or CD8 allow the identification of T-cells. Of course, the cell-directed additional detectable markers should be detectable separately from the detectable markers which are able to bind with the target analyte. For example, they should exhibit a different fluorescence wavelength.

Regarding the paramagnetic particles, paramagnetic particles of a same type may be used, the surface of which is coated with different capture elements. The set of these capture elements at the surface of each paramagnetic particle, allows a binding with all the target analytes. According to another embodiment, a mixture of paramagnetic particles of several types may be used. The surface of each paramagnetic particle may be coated with a specific capture element capable of binding with a specific target analyte. The set of paramagnetic particles used in such a case preferably allow binding with all the target analytes.

As seen in **Fig. 1****,** the functionalized paramagnetic particles **22',** detectable markers **24** and biological cells are mixed in a suspension fluid **20,** preferably a liquid, typically an aqueous liquid, more typically a liquid culture medium. From this suspension liquid, droplets **18** are formed.

*Jöensson et al., 2012* summarizes various techniques for producing droplets according to the invention. *Eyer et al., 2017* discloses more precisely such a method. Preferably the droplets are produced as an emulsion of droplets of the suspension fluid, typically an aqueous liquid culture medium **20,** in a dispersion medium, typically an oil-based fluid **32,** preferably as a homogenous monodisperse emulsion of aqueous liquid droplets **18** in an oil-based liquid **32.**

In the invention, an assay droplet has a volume which is typically below 200 picoliters (pL). Typically, the preferred volume for an assay droplet **18** is comprised between 4 pL and 600 pL, preferably within the range from 30 pL to 100 pL. In the exemplary application, the volume of the assay droplets **18** was about 50 picoliters.

This step of droplet formation, where assay droplets **18** are formed from the suspension fluid containing paramagnetic particles **22, 22',** detectable markers **24,** and at least one biological cell **10,** is often called encapsulation, because the paramagnetic particles, detectable markers, and biological cell(s) are encapsulated inside the assay droplet **18.**

As shown very schematically in **Fig. 1****,** encapsulation may involve flowing a solution of the suspension fluid, incorporating the functionalized paramagnetic particles **22',** detectable markers **24** and biological cells, in a microchannel, for example capillary tube **34,** towards a cross-junction **36** with another microchannel, for example a capillary tube **38** in which circulates a flow of the dispersion medium **38.** By proper calibration, an emulsion of monodisperse droplets of the suspension fluid in the dispersion fluid is collected in a downstream output capillary tube **40.**

Such microfluidic droplet formation techniques are known in the art. The fabrication and operation of a microfluidic chip for droplet production has been previously described in *Mazutis et al., 2013.* The microfluidic system has been optimized to co-encapsulate a single mammalian cell and reagents in one droplet. The device may in fact contain three inlets for bringing respectively (I) the dispersion fluid, (II) the suspension of cells in the suspension fluid and (III) the assay reagents, which may include functionalized paramagnetic particles, detectable markers, and possibly other reagents, such as live/dead cell probes, stimulating agents, etc.... The device has, as described above, at least one outlet for collecting the produced droplets.

In the devices as described above, assay droplets are formed at the flow-focusing junction **36.** Preferably the device and the flows in the device are calibrated so the flows at the junction **36** are laminar.

In order to prevent clogging of the microfluidic device during experiments, passive filters may be added at one or several of the inlets, or at each inlet. Fluid resistors and/or serpentine channels may be used to damp fluctuations of the flow within the microchannels. Such a droplet generating device produces highly monodisperse and stable aqueous droplets in inert fluorinated oil over extended periods of time.

The process may be devised such that an assay droplet **18** comprises other reagents. For example, assay droplets may include an active agent in the form of a stimulation agent for stimulating the activity of the cell, preferably stimulating especially the biological process from which the target analyte originates. In the exemplary application, where the bioassay is devised to monitor TNF-alpha secretion by a single monocyte, the stimulation agent was lipopolysaccharide (LPS). Using a three inlet device as described just above, the contact between cell and assay reagents occurs only after encapsulation in the assay droplet **18.** Therefore, behavior of cell dynamics is observed only after stimulation within the assay droplet **18.**

In order to achieve monodispersity of the functionalized paramagnetic particles it can be necessary to prevent them from aggregating. One way to prevent this can include sonicating the functionalized paramagnetic particles **22'** for 1 to 3 minutes in a water bath sonicator prior to encapsulation. Together with the use of nanoparticles as paramagnetic particles, this has proven to notably improve the performance of the process, as each individual cell is encapsulated within an assay droplet with the same amount of functionalized paramagnetic particles, and the formation of droplets containing a cell and no functionalized paramagnetic particles is avoided.

As in the know prior art, the process includes storing several assay droplets **18** along a single layer along a horizontal storage plane, here in a storage chamber **42.**

In the shown embodiment, the storage chamber **42** is formed in a sample carrier device **44.**

In the example, depicted in **Figs. 4, 5** and **6****,** the storage chamber **42** is designed to be transparent in a detection process of the detectable markers **24.** In the example, the detection process is an optical detection process. Therefore, the storage chamber **42,** and in the example, the part of the sample carrier device **44** which forms the storage chamber **42,** is optically transparent, at least at the wavelengths involved in the detection process, which may include wavelengths in the visible domain, and/or wavelengths in the infrared domain and/or in the ultraviolet domain.

In the shown example, the sample carrier device **44** comprises two plates, namely a top plate **46** and a bottom plate **48,** which extend parallel to each other. The plates **46, 48** can be made of glass, or of similar material, for example a transparent polymer material. In operation of the apparatus, at least during the step of detection of the detectable markers which will be discussed below, the two glass plates **46, 48** are horizontal. They form the two top and bottom walls of the storage chamber **42.** In the exemplary application, the plates were plates of 75 millimeters long by 25 millimeters large.

In the exemplary application, the two glass plates **46, 48** are affixed one to the other by an adhesive film **50,** preferably having adhesive on both sides, which is sandwiched between the two glass plates. The thickness of the adhesive film **50** determines vertically the height of the storage chamber. Indeed, the storage chamber **42** is formed between two parallel top **47** and bottom **19** inner wall surfaces, which are respectively the bottom surface of the top plate **46** and the top surface of the bottom plate **48.** The inner wall surfaces **47, 49** extend, in operation of the apparatus, at least during the detection phase of the process, parallel to a horizontal plane and, they are separated vertically by a height, which is the height of the storage chamber **42.**

The height of the storage chamber **42** is designed to make sure that the stored assay droplets are stored along a single layer that layer extending along a horizontal plane, here parallel to the plates **46, 48.** Thus the height of the storage chamber is designed according to the volume of assay droplets **18.** The height of the storage chamber **42** is preferably less than 2 times, preferably less than 1.5 times the diameter of a sphere having the same volume as the assay droplets **18.** On the other hand, the height of the storage chamber is preferably more than 0.5 times, preferably more than 0.8 times the diameter of a sphere having the same volume as the assay droplets **18.** Typically, for a volume of the assay droplets comprised between 4 picoliters (pL) and 600 picoliters (pL), the height of the storage chamber may be between 10 micrometers (µm) and 200 micrometers (µm).

As visible in **Fig.** 5, the adhesive film **50** comprises an internal cutout **52,** the border **54** of which delimits the extension of the storage chamber along the horizontal plane. In the exemplary application, an about 40-50 micrometers thick double-sided adhesive film **50** was used to bind the two plates **46, 48** together, by applying moderate pressure with clamps, followed by 1-hour incubation at 90 °C. Studies have demonstrated that the bond strengths obtained with double-sided adhesive film is reproducible and comparable to that of plasma and corona discharge. Several double-sided adhesive films with different thickness are commercially available (for example from 3M®) and any shape and size of the storage chamber can be obtained by cutting the internal cutout **52** at a desired shape and size in the film with a simple knife plotter or with a laser cutting machine. Adhesive films have a strong adhesion to glass surfaces and are stable at high temperature, thus preventing fluid leakage and water loss from the emulsion. Polyester double-sided adhesive films have proven to provide robust assembly of glass plates and accurate control of height of the storage chamber **42.**

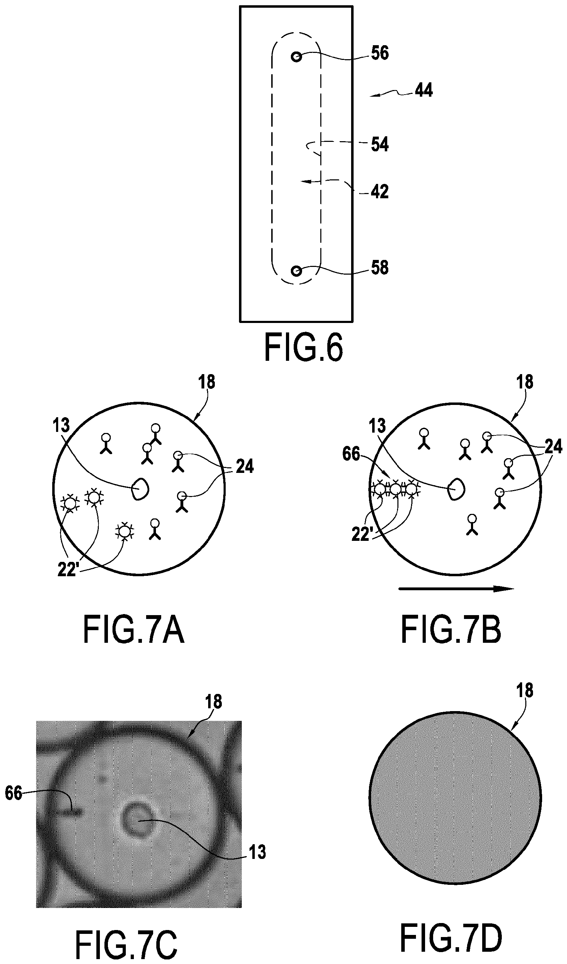

Also shown on **Figs. 5** and **6** is the presence of at least one inlet port for the storage chamber. In the shown embodiment, the sample carrier device **44** comprises two ports, an inlet port and a vent port, which both give access to the storage chamber **42.** **Figs. 5** and **6** show two holes **56, 58** made in one the plates, here the top plate **46,** in correspondence with the internal cutout **52,** so that the holes provide access to the storage chamber **42.** One hole **56** may be used as an inlet port, while the other hole **58** may serve as a venting port, to ease the filling of the storage chamber by allowing trapped air, or excess of suspension, to escape from the storage chamber **42** when a suspension containing assay droplets is introduced through the inlet port. The ports are preferably equipped with connectors on the external side (not shown in the Figures).

The process includes detecting, within a given stored assay droplet, a physical characteristic of the detectable markers contained in the given assay droplet, by optical imaging of the detectable markers. This step is referred to as the detection phase of the method. This step of detecting, and the detection as a whole may include, or may consist in, a mere analysis of presence or absence of a given physical characteristic in an image, or an analysis of its presence in an image above and/or below a threshold, which may be predetermined or not; or within a range, predefined or not. The steps of detecting may comprise the evaluation of the value of the physical parameter, including in view of dosing the physical characteristic, and thus of dosing the underlying quantity of target analyte.

To that effect, the apparatus shown in **Fig. 3** includes a detector **60** of a physical characteristic of the detectable markers **24** contained in a given assay droplet **18** stored in the storage chamber **42.** The detector **60** may be part of a detection station **62** of the apparatus. Typically, the detector **60** comprises at least one camera which is able to take pictures of the assay droplets **18** stored in the storage chamber **42.** The camera views the storage chamber **42** along an optical imaging axis **A1** which is perpendicular to the horizontal plane, thus vertical. Optical imaging may be performed from below the storage chamber **42,** i.e. through its bottom plate **48.** Of course, the detector **60** may comprise lenses and/or mirrors such that the camera itself may be arranged with a different orientation, not necessarily below the storage chamber **42** at the detection station **62.** The detection station comprises several lights for illuminating the storage chamber. The lights may have different illumination wavelengths, with for example one or several lights having a monochromatic illumination wavelength, and/or one or several lights having a limited illumination wavelength bandwidth, for example limited to a part of the ultraviolet spectrum or to a part of the infrared spectrum, and/or one or several lights having a broad illumination wavelength bandwidth, for example covering the visible spectrum or part thereof to achieve a white light illumination. The lights may be selectively activated to achieve different illumination conditions. Among these lights that may be selectively activated, some are able to stimulate the detectable markers and, optionally, the vital dye(s) and/or the non-vital dye(s) that are used to carry out the process according to the invention.

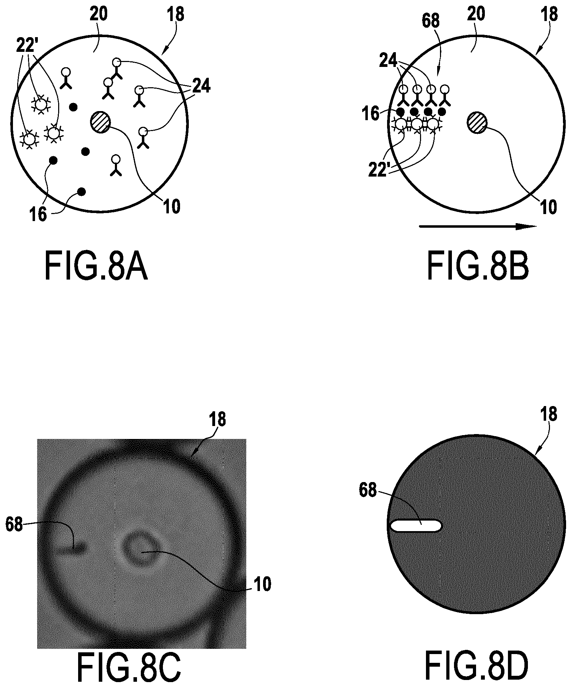

The camera may typically include an imaging array sensor, such as a CCD array sensor or a CMOS array sensor where the imaging array sensor comprises an array of sensor element. The camera operates an optical imaging process by forming an optical image of the storage chamber on the imaging array sensor. The imaging array sensor delivers an image of the storage chamber where the detectable markers may be visible, under certain conditions which are explained below in relation to **Figs. 7A** to **7D** and **8A** to **8D.** The image can be a digital signal. As part of the detection phase, the image can be analyzed by a human operator or by an automated process, such as a computer image analysis process. Such analysis may involve identifying and counting the number of cells, aggregates, etc..., in one or several images of the storage chamber, and may involve comparing such data between images of the same assay under different illumination conditions.

In an exemplary application, the detector comprises a monochromatic camera which delivers a greyscale digital image of its field of view. In the image delivered by the monochromatic camera one point of an object in the camera's focusing plane corresponds to one single point in the image, where the single point has a grey level representative of the intensity of the light received by the corresponding sensor element.

In some embodiments, the detection phase involves detecting fluorescence of fluorescent detectable markers. With a fluorescent detectable marker, the physical characteristic of the detectable markers which is detected by the detector is the fluorescence light emitted by the marker when it is properly excited. To that effect, the detector, typically the imaging array sensor discussed above, must be sensitive in a wavelengths bandwidth containing the fluorescence wavelength(s) of the detectable marker. The detector may comprise one or several optical bandwidth filter(s) which can be selectively activated and deactivated in accordance with the detectable marker(s) and, optionally, with the vital dye(s) and/or the non-vital dye(s) that are used to carry out the process according to the present invention, for optically imaging the storage chamber. Different optical filters may therefore be used, for the acquisition of different images, for detecting different markers, optionally, different vital or non-vital dyes emitting different fluorescence wavelengths

The detection phase may also comprise acquiring, with the detector **60,** a bright-field image.

In some embodiments, the detector **60** can perform imaging of the entire storage chamber **62** in one single frame, corresponding to the field of view of the detector or to part of it. However, the field of view of the detector **60** may be smaller than the extension of the storage chamber **42,** or smaller than a region of interest of the storage chamber. In such a case, the process and apparatus may be devised such that several juxtaposed frames are taken, each frame being an image of a portion of the region of interest, with a relative displacement between the storage chamber and the detector between each frame.

In the exemplary application aiming at tracking the behavior of cells when trapped within assay droplets, a region of interest having an overall square area of 220 square millimeters of a monolayer array of such assay droplets, immobilized in the storage chamber, was imaged by the detector. In the experimental setup for the exemplary application, the sample carrier device **44** having the storage chamber **42** was placed on the motorized stage of an inverted epifluorescence microscope (Nikon®). Both the microscope and the sample carrier device **44** having the storage chamber **42** were encompassed in a box at 37 °C. A 10X magnification objective (CFI Plan Apochromat Lambda 10X, Nikon, N.A.=0.45, working distance 4 mm) was used to image 125 joint frames, of 2048x2048 pixels, in 5 different imaging channels, corresponding to 5 different imaging wavelength bandwidths. Focus was manually set on the first field so that the contours of droplets and the paramagnetic particles appear as dark objects on a clear background in a bright-field image.

The magnification used for image acquisition during the detection phase can also be a limiting factor of the procedure. For example, a 10X magnification can be a compromise between scanning time and resolution, as it enabled imaging simultaneously about 600 droplets per frame with a sufficient resolution so that all the relevant objects within the image (i.e. droplets and encapsulated cells and marked and magnetized combinations) could be discriminated during an automated image processing.

As both automated object detection and fluorescence intensity measurements rely on focus quality, control of focus drift throughout the imaging procedure (of a large area and over a long duration) is crucial. Mechanical effects, vibrations, thermal changes and droplet movement can cause the focus to drift. Droplet coalescence is also a factor that can affect focal plane of the droplet array. This limitation may be addressed by using manual focus or automatic focus. However, this limitation was preferably addressed in the exemplary application using the Perfect Focus System (PFS) of the Nikon® microscope to provide fast and efficient real-time adjustment of the focal plane during the long-lasting acquisitions and surface scanning.

However, such focusing may not be helpful if different elements in a same picture frame are not in the same focal plane.

The known process includes the generation of a magnetic field through the stored assay droplets for spatially arranging the paramagnetic particles inside the stored assay droplets. Therefore the apparatus includes a magnetic field generator creating a magnetic field across the storage chamber, for spatially arranging the paramagnetic particles inside the assay droplets stored in the storage chamber. Indeed, by providing such a magnetic field, all the paramagnetic particles contained in one assay droplet are thus gathered in one region of the droplet which depends on the orientation of the magnetic field at the location of the droplet.

The effect of the magnetic field is exemplified in schematics shown respectively in **Figs. 7A, 7B, 7C, 7D** and **8A, 8B, 8C****,** **8D****.** **Figs. 7A** and **8A** are a representation of an assay droplet **18** containing functionalized paramagnetic particles **22'** and detectable markers **24,** in the absence of any magnetic field. All these elements are thus randomly dispersed within the droplet **18.** In **Fig. 7A****,** no target analyte is present, whereas the case of presence of target analytes corresponds to **Fig. 8A****.** In reality, the presence of target analytes, which are supposed to be produced by a non-shown biological cell, would lead to the formation of marked and magnetized combinations, as shown in **Fig. 8B****.**

**Fig. 7B** and **Fig. 8B** depict the assay droplets **18** in presence of a magnetic field according to the invention. Due to this magnetic field, the paramagnetic particles **22'** are gathered in one region of the assay droplet **18,** forming an aggregate **66, 68** of paramagnetic particles **22'.** In the schematics, the paramagnetic particles are shown to be arranged, under the influence of the externally applied magnetic field, in a rod-like arrangement, but other shapes are possible for the aggregate **66, 68.** However, in **Fig. 7A** is shown that, in the absence of a target analyte, the detectable markers do not bind to the functionalized paramagnetic particles **22',** or at least not to any significant level. Thus, even though the paramagnetic particles **22'** are aggregated in one region of the droplet, the detectable markers **24** remain randomly dispersed and not show any relocation of the detectable marker on the aggregated paramagnetic particles. In **Fig. 7B****,** without the presence of target analytes in the assay droplet, it is shown that an aggregate **66** of functionalized paramagnetic particles **22'** may form, but such aggregate **66** does not contain detectable markers, at least not in any significant quantity, and can thus be called a non-marked aggregate **66.**

In **Fig. 7C** is shown a bright-field image that can be obtained with the process according to the invention. As in this example shown in **FIG. 7C****,** it is possible that paramagnetic particles are visible in a bright-field image, thanks to the size of the individual paramagnetic particles and/or to the size of the aggregate **66** formed when under the influence of the externally applied magnetic field. As shown schematically on **Fig. 7D****,** under an excitation light of a specific wavelength able to stimulate the detectable markers **24,** the droplet **18** remains uniformly and weakly fluorescent, because the detectable markers, now fluorescent, remain disseminated/dispersed in the entire droplet. The non-marked aggregate **66** is not visible under such lighting.

To the contrary, **Fig. 8B** illustrates that, in the presence of target analytes, which have caused the formation of marked and magnetized combinations **25,** the aggregate **68** formed by the paramagnetic particles under the influence of the magnetic field causes an aggregation, in the same aggregate **68,** also of detectable markers **24.** Such an aggregate can be called a marked aggregate **68.** As illustrated in **Fig. 8C****,** such marked aggregate **68** would possibly be visible in a bright-field image, similarly to the case of **Fig. 7C****,** but more importantly now the detector **60** is capable of imaging, as illustrated in **Fig. 8D** the presence of the detectable markers in the marked aggregate, thanks to the aggregation, thus to the localized higher concentration of the detectable markers. When submitted to an excitation light of a specific wavelength able to stimulate the detectable markers **24,** the droplet **18** appears with a fluorescent-marked aggregate **68.** Indeed, the detectable markers, fluorescent under the excitation, are visibly relocated and concentrate on the aggregate **68,** and the rest of the droplet 18 appears darker

However, according to the invention, the process and apparatus are devised in a way that the magnetic field, which is generated across the storage chamber, and thus across the assay droplets contained in the storage chamber, is such that the paramagnetic particles are attracted towards a lateral and inferior portion of the given stored assay droplet in which they are contained. This is achieved thanks to the magnetic field generator which generates a magnetic field such that the paramagnetic particles are attracted horizontally towards at least one side of the storage chamber and vertically towards the bottom inner wall surface **49** of the storage chamber **42.** The terms "inferior", "bottom" and "below" are used herein with reference to the orientation of gravity, the orientation of which also determines the horizontal plane directions.

Indeed, tests have shown that, during the detection phase, a biological cell contained in a given assay droplet **18** tends to be located at the center of the droplet, when viewed along a vertical optical viewing axis, and towards the bottom of the droplet, by the mere action of gravity on the cell and of the shape of the droplet.

To the contrary, thanks to the properly arranged magnetic field, the aggregate, which may contain detectable markers bound to the paramagnetic particles, is attracted horizontally towards the side of the given droplet. Thus, by being to the side, the aggregate of detectable markers is, with respect to the optical viewing axis, laterally offset in a horizontal plane from the biological cell contained in the given droplet. This avoids or limits the risk that the aggregate and the cell are wholly or partially hidden of view one by the other along the vertical optical imaging axis **A1.**

Moreover by generating a magnetic field which attracts the paramagnetic particles towards the bottom of the storage chamber, thus towards the bottom of the assay droplet **18,** it is obtained that the paramagnetic particles, including in the form of a marked aggregate **68,** are located, along the optical viewing axis **A1**, in a same horizontal plane as the biological cell **10** contained in the same droplet, or with at least with a very little distance along the optical viewing axis. This ensures that, despite a possible very shallow depth of field, an image of the assay droplet **18** may show both the biological cell **10** and the paramagnetic particles, in the form of a marked aggregate **68,** with comparable sharp focus. Such sharp focus of both elements will greatly facilitate identification of both elements, including by automatic computerized shape recognition, and will in parallel allow an accurate of determination of the physical characteristic which is to be detected from the detectable markers.

Therefore, the paramagnetic particles are attracted towards a lateral and inferior portion of the assay droplet, and finally find themselves located side by side with the cell which has fallen to the bottom of the assay droplet, under a phenomenon of sedimentation.

In reference with **Fig. 7C** and **Fig. 7D****,** the absence of TNF-alpha secretion is marked by an absence of relocation and concentration of the fluorescence. In the assay droplet, the non-marked aggregate **66** and the biological cell **13** are located side by side, and both appear clearly on the bright-field image. The absence of TNF-alpha secretion is thus not due to a lack of cell encapsulation. An additional use of a vital dye and/or a non-vital dye would make it possible to check the cell viability.

In this regard, fluorogenic mitochondrial stains such as the ones belonging to the MytoView™ range are examples of vital dyes particularly useful to label live cells. CellTiter-Blue® Cell Viability Assay, from Promega Corporation, 2800 Woods Hollow Road, Madison, WI 53711-5399 USA may be used also to label live cells. On the other hand, cell membrane-permeable fluorogenic caspase substrates such as the ones belonging to the NucView® range allow a detection of apoptosis and are therefore particularly interesting examples of non-vital dyes to label dead cells. MultiTox-Fluor Multiplex Cytotoxicity Assay, from Promega Corporation, may be used to measure the number of live and dead cells simultaneously. HCS DNA damage Kit (H10292), from Thermo Fisher Scientific Inc., may also be used.

In reference with **Fig. 8C** and **Fig. 8D****,** a relocation and concentration of the fluorescence are observed. A secretion of TNF-alpha is detected. In the assay droplet, the marked aggregate **68** and the biological cell are located side by side; they both appear clearly on the bright-field image. This allows to check that only one cell has been encapsulated in the assay droplet, and the recorded fluorescence intensity that is correlated with the quantity of secreted TNF-alpha is actually linked to the activity of a single cell. An additional use of a vital dye and/or a non-vital dye would make it possible to check the cell viability.

In some cases, several cells can be encapsulated in one same assay droplet. The process and apparatus according to the invention allow a following up of each assay droplet, in time. Therefore, it can be checked out whether several cells were co-encapsulated from the beginning at the encapsulation stage, or do come from a cell division occurring during the assay.

In some embodiments, the magnetic field is such that the paramagnetic particles are attracted towards a similarly disposed lateral and inferior portion of all the stored assay droplets. This is typically achieved with magnetic field generator which generates a magnetic field such that the paramagnetic particles are attracted horizontally towards a same side of the storage chamber.

Typically, such magnetic field can be asymmetric with respect to the stored assay droplets. This may be achieved by using an apparatus in which the magnetic field generator comprises one or several magnets, especially one or several permanent magnets or electromagnets, forming a magnet assembly **70** which is arranged horizontally asymmetrically with respect to the storage chamber, and below the level of the storage chamber **42.**

It can be noted that, preferably, optical imaging may be performed from the side of the storage chamber **42** along the vertical axis towards which the aggregate is attracted by the magnetic field, thus for example from below the storage chamber **42,** i.e. through its bottom plate **48.** Indeed, because the cells tend to be attracted towards the bottom plate, this allows minimizing the thickness of any suspension fluid or dispersion fluid in the light path for the imaging process.

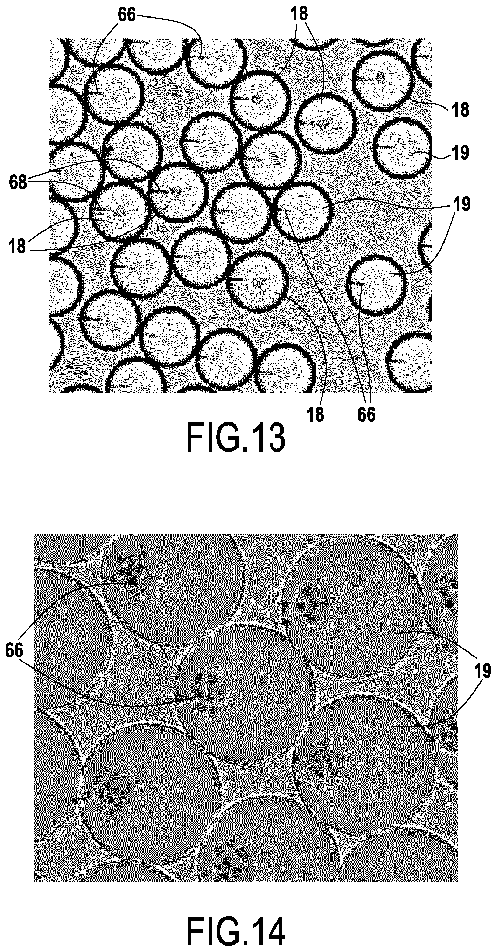

Overall, the process and apparatus according to the invention allow obtaining images such as shown in **Fig. 13** of several droplets in a storage chamber. The image of **Fig. 13** is a bright-field image showing droplets **19** without any cells, and assay droplets **18** containing cells. In all the droplets, an aggregate is clearly visible, perfectly in focus and located towards a side of the droplet. In assay droplets **18** containing cells, both the cell and the aggregate is clearly visible, perfectly in focus, and well separated one from the other. This ensures optimal and reliable detection of both the cell and the aggregate via known image analysis techniques.

It is to be noted that in the below described examples, the storage chamber **42** is part of the sample carrier device **44** and the magnetic field generator **70** is affixed to said sample carrier device. However, the magnet assembly does not need to be affixed to the sample carrier **44.** The application of a magnetic field is especially important during the detection phase of the process, i.e. when the storage chamber is in front of the detector **60.** Therefore, the magnetic field generator may be independent of the sample carrier device **44.** The magnetic field generator can be part of the detection station **62** of the apparatus. Indeed, as the storage chamber **42** is for example part of the sample carrier **44** device, the apparatus may be designed such that the sample carrier device **44** is movable relative to the detection station **62** comprising the detector 60. The sample carrier device **44** can thus be brought, manually or automatically, in coincidence with the detection station **62** for the performance of the detection phase. In such a case, it can be provided that the magnetic field generator **70** can be fixed with respect to the detection station **62,** especially fixed with respect to the detector **60.** It is then preferable that, for the performance of the detection phase at the detection station, the storage chamber **42** is always brought into a predefined position with respect to station **62,** especially with respect to the detector **60.**

In the case shown in **Figs. 4, 5** and **6****,** the magnet assembly **70** comprises only one magnet **71,** which is here a permanent magnet but which could also be an electromagnet. The magnet **71** exhibits a north pole N and a south pole S. The magnet is for example shaped as a parallelepiped. The magnet is for example arranged to extend along one side of the storage chamber **42.**

In the example, the storage chamber **42** is elongated along a first direction of the horizontal plane. The magnet assembly, for example the single magnet **70,** may thus be placed on a side of the storage chamber, transversally offset horizontally along a second direction, which is perpendicular to the first direction, compared to said side of the storage chamber.

In the shown example, the magnet assembly **70,** for example the single magnet **71,** may be entirely to the side of the storage chamber **42.** In such a case, both of the poles of the magnet assembly are located horizontally to said side of the storage chamber. In such a case, the magnetic field is such that the paramagnetic particles are attracted, along the second direction, horizontally towards a side of the storage chamber.

It is to be noted that, in some embodiments, the assay droplets are preferably not able to move inside the storage chamber during the detection phase, i.e. when the magnetic field is applied. This may be achieved by substantially filing entirely the storage chamber with such assay droplets which are thus arranged contiguously over the whole extension of the storage chamber in the horizontal plane.

However, in other embodiments, the droplets may be able to move within the storage chamber. In this case, especially in the case where the magnetic field generator is not affixed to the storage chamber but pertains rather to the detection station, the application of the magnetic field may result in an initial movement of the magnetic field, due to the attraction of the paramagnetic particles contained in the droplets which may cause the movement of the droplets. However, even in that case, any movement of the droplets stops once the droplets have gathered on a corresponding side of the storage chamber.

To make sure that the paramagnetic particles are attracted towards an inferior portion of the stored assay droplets, the magnet assembly **70** is arranged below the level of the bottom inner wall surface **49** of the storage chamber **42.** In the shown example, the bottom inner wall surface **49** of the storage chamber pertains to a bottom plate **48** of the storage chamber, for which it is a top surface. The bottom plate **48** exhibits a bottom external wall surface **51** opposite its inner wall surface **49.** The magnet assembly is preferably arranged below the level of the bottom external wall surface **51.**

In a non-depicted embodiment, it also could be provided that the paramagnetic particles are attracted towards a similarly disposed first lateral and inferior portion of a first group of the stored assay droplets, and towards a similarly disposed second lateral and inferior portion of a second group of the stored assay droplets, the first and second lateral and inferior portions being differently located in the respective assay droplets.

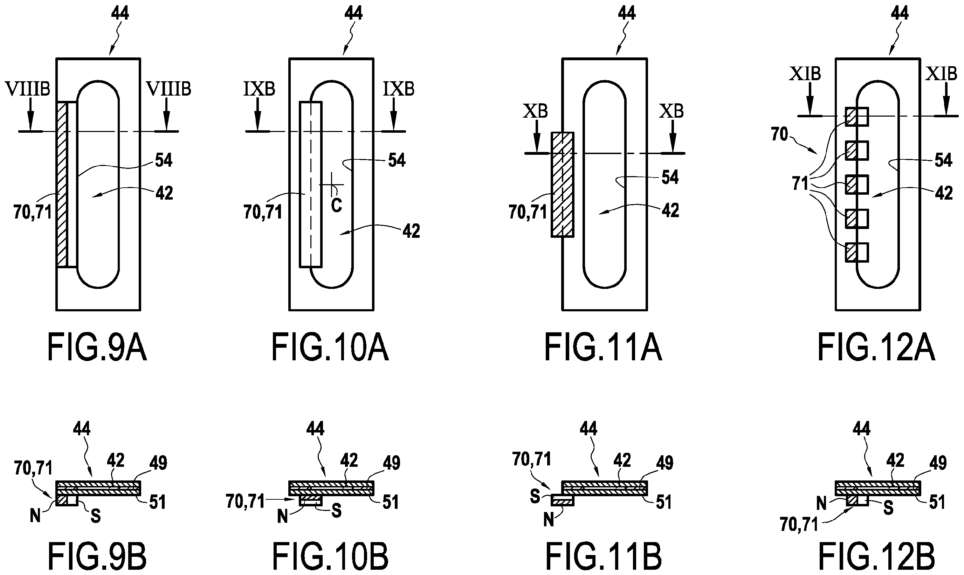

In the embodiment of **Figs. 4** to **6****,** the north pole N of the magnet assembly is on a top side of the magnet assembly **70,** while the south pole S in on a bottom side of the magnet assembly, the magnet having thus a vertical magnetic moment. However, an inverted configuration is possible with the north pole N on top, as in the embodiment of **Figs. 11A** and **11B****,** with still exhibits a vertical magnetic moment. In both cases, this would result in the aggregate being in the shape of rod-like elements in the horizontal as shown in **Figs. 7B, 7C****,** **8B, 8C****,** **8D** and **13****.**

In the embodiment of **Figs. 9A** and **9B****,** the north pole N of the magnet assembly is on one lateral side of the magnet assembly **70,** while the south pole S in on the opposite lateral side of the magnet assembly according to the horizontal direction, thus having a horizontal magnetic moment. In such a case, this would result in the aggregate being as shown in **Fig. 14** in a horizontal plane.

In some embodiments, such as those of **Fig. 4** and of **Fig. 4B****,** the side of the storage chamber **42** is defined as anything which is laterally offset with respect to a periphery of the storage chamber along the horizontal plane, for example the contour **54** of internal cut-out **52.** In this case, the magnet assembly **70** does not overlap with the extension of the storage chamber **42.**

In some embodiments, such as the embodiment of **Figs. 10A** and **10B****,** the side of the storage chamber **42** is defined as anything which is laterally offset with respect to a centroid C of the extension of the storage chamber **42** along the horizontal plane. In this case, the magnet assembly **70** may overlap, but only partially, with the extension of the storage chamber **42.**

In the previous examples, the magnet assembly **70** is continuous along one side of the storage chamber, over the entire extension of said side of the storage chamber. However, as in the example of **Figs. 11A** and **11B****,** the magnet assembly may extend along only a part, less than the entirety, of the extension of the side of the magnet assembly. Moreover, the magnet assembly may be discontinuous, for example comprising several magnets which are spaced one from the other. **Figs. 12A** and **12B** show such arrangement, with several magnets spaced one from the other, in this case with magnets having a horizontal magnetic moment, and with magnets which partially overlap with the extension of the storage chamber. However, other configurations could have several spaced magnets with vertical magnetic moment, and/or several spaced magnets not overlapping with the extension of the storage chamber.

## Claims

1. Microfluidic droplet-based assay process for analyzing biological cell activity by detection of at least one target analyte (**16**) contained in one or several droplets, wherein the process comprises the step of creating several assay droplets (**18**) of a liquid culture medium where an assay droplet contains:
- paramagnetic particles (**22**, **22**');
- detectable markers (**24**);
- and, at least one biological cell (**10**) potentially capable of releasing at least one target analyte (**16**); wherein the paramagnetic particles (**22**, **22**') and the detectable markers (**24**) are able to bind with a target analyte (**16**) to form a marked and magnetized combination (**25**) with a target analyte (**16**),
wherein the process includes storing several assay droplets along a single layer along a horizontal storage plane,
wherein the process includes the generation of a magnetic field through the stored assay droplets for spatially arranging the paramagnetic particles inside the stored assay droplets,
and wherein the process includes detecting, within a given stored assay droplet, a physical characteristic of the detectable markers contained in the given assay droplet, by optical imaging of the detectable markers, **characterized in that** the generation of a magnetic field generates a magnetic field such that the paramagnetic particles are attracted towards a lateral and inferior portion of the given stored assay droplet in which they are contained.

2. Microfluidic droplet-based assay process according to claim **1, characterized in that** the magnetic field is such that the paramagnetic particles (**22**, **22**') are attracted towards a similarly disposed lateral and inferior portion of all the stored assay droplets.

3. Microfluidic droplet-based assay process according to any preceding claim, **characterized in that** the magnetic field is asymmetric with respect to the stored assay droplets.

4. Microfluidic droplet-based assay process according to any preceding claim, **characterized in that** it comprises detecting fluorescence of fluorescent detectable markers and/or detecting radioisotope-labeled detectable markers.

5. Microfluidic droplet-based assay process according to any preceding claim, **characterized in that** it comprises providing, in the assay droplet, additional detectable markers able to bind with specific proteins expressed on the biological cell (**10**) surface to tag the biological cell (**10**).

6. Microfluidic droplet-based assay process according to any preceding claim, **characterized in that** the liquid culture medium comprises at least one vital dye and/or at least one non-vital dye.

7. Apparatus for a microfluidic droplet-based assay process for analyzing biological cell activity by detection of at least one target analyte (**16**) contained in one or several droplets, wherein the apparatus comprises a storage chamber (**42**) for storing several assay droplets (**18**) of a liquid culture medium containing :
- paramagnetic particles (**22**, **22**');
- detectable markers (**24**);
- and, at least one biological cell (**10**) potentially capable of releasing at least one target analyte (**16**);
where the storage chamber is transparent in a detection process of the detectable markers and where the storage chamber is formed between two parallel top and bottom inner wall surfaces (**47**, **49**) of the storage chamber, the inner wall surfaces extending parallel to a horizontal plane and being separated vertically by a height, such that the assay droplets are stored along a single layer;
wherein the apparatus includes a magnetic field generator (**70**, **71**) for spatially arranging the paramagnetic particles inside the assay droplets stored in the storage chamber,
and wherein the apparatus includes a detector (**60**) of a physical characteristic of the detectable markers contained in a given assay droplet stored in the storage chamber,
**characterized in that** the magnetic field generator generates a magnetic field such that the paramagnetic particles are attracted horizontally towards at least one side of the storage chamber and vertically towards the bottom inner wall surface of the storage chamber.

8. Apparatus according to claim 7, **characterized in that** the magnetic field generator comprises one or several magnets (71) forming a magnet assembly (70) which is arranged below the level of the bottom inner wall surface (**49**) of the storage chamber.

9. Apparatus according to claim **7** or **8, characterized in that** the inner bottom wall surface (**49**) of the storage chamber pertains to a bottom wall (**48**) of the storage chamber which exhibits a bottom external wall surface (**51**) opposite its inner wall surface, and **in that** the magnet assembly is arranged below the level of the bottom external wall surface (**51**).

10. Apparatus according to any of claims **7** to **9, characterized in that** the magnetic field generator (**70**, **71**) generates a magnetic field such that the paramagnetic particles are attracted horizontally towards a same side of the storage chamber (**42**).

11. Apparatus according to any of claims **7** to **10**, **characterized in that** the magnetic field generator comprises one or several magnets (**71**) forming a magnet assembly (**70**) and having each magnetic poles, wherein at least one of the poles of each of the magnets is located horizontally to a same side of the storage chamber (**42**).

12. Apparatus according to any of claims **7** to **10**, **characterized in that** the magnetic field generator comprises one or several magnets (**71**) forming a magnet assembly (**70**) and having each magnetic poles, wherein both of the poles of the one or several magnets are located horizontally to a same side of the storage chamber.

13. Apparatus according to any of claims **7** to **12, characterized in that** the storage chamber (**42**) is part of a sample carrier device (**44**) and **in that** the magnetic field generator (**70**, **71**) is affixed to said sample carrier device.

14. Apparatus according to any of claims **7** to **12**, **characterized in that** the magnetic field generator (**70**, **71**) is part of a detection station (**62**) of the apparatus and **in that** the storage chamber (**42**) is part of a sample carrier device (**44**), the detection station and the sample carrier device being movable relative one to the other and being brought into coincidence for detection of at least one target analyte contained in one or several droplets contained in the storage chamber of the sample carrier device.

15. Apparatus according to any of claims **7** to **14**, **characterized in that** the sample carrier device (**44**) comprises two plates (**46**, **48**) forming the two walls of the storage chamber (**42**), and **in that** the two plates are affixed one to the other by an adhesive film (**50**) sandwiched between the two plates, wherein the thickness of the adhesive film determines vertically the height of the storage chamber.

16. Apparatus according to claim **15, characterized in that** the adhesive film (**50**) comprises an internal cutout (**52**), the border (**54**) of which delimits the extension of the storage chamber (**42**) along the horizontal plane.

## Patentansprüche

1. Mikrofluidischer tröpfchenbasierter Assay-Prozess zum Analysieren einer biologischen Zellaktivität durch einen Nachweis von mindestens einem Zielanalyten (16), der in einem oder mehreren Tröpfchen enthalten ist, wobei der Prozess den Schritt des Erzeugens mehrerer Assay-Tröpfchen (18) eines flüssigen Kulturmediums umfasst, wobei ein Assay-Tröpfchen enthält:
- paramagnetische Partikel (22, 22'),
- nachweisbare Marker (24),
- und mindestens eine biologische Zelle (10), die möglicherweise dazu in der Lage ist, mindestens einen Zielanalyten (16) freizusetzen, wobei die paramagnetischen Partikel (22, 22') und die nachweisbaren Marker (24) dazu in der Lage sind, an einen Zielanalyten (16) zu binden, um mit einem Zielanalyten (16) eine markierte und magnetisierte Kombination (25) zu bilden,
wobei der Prozess das Speichern von mehreren Assay-Tröpfchen entlang einer einzigen Schicht entlang einer horizontalen Speicherebene beinhaltet,
wobei der Prozess das Generieren eines Magnetfelds durch die gespeicherten Assay-Tröpfchen zum räumlichen Anordnen der paramagnetischen Partikel im Inneren der gespeicherten Assay-Tröpfchen beinhaltet,
und wobei der Prozess das Nachweisen, innerhalb eines gegebenen gespeicherten Assay-Tröpfchens, einer physikalischen Eigenschaft der nachweisbaren Marker, die in dem gegebenen Assay-Tröpfchen enthalten sind, durch eine optische Abbildung der nachweisbaren Marker beinhaltet,
**dadurch gekennzeichnet, dass** das Generieren eines Magnetfelds ein Magnetfeld generiert, sodass die paramagnetischen Partikel zu einem seitlichen und unteren Teil des gegebenen gespeicherten Assay-Tröpfchens angezogen werden, in dem sie enthalten sind.

2. Mikrofluidischer tröpfchenbasierter Assay-Prozess nach Anspruch 1, **dadurch gekennzeichnet, dass** das Magnetfeld dergestalt ist, dass die paramagnetischen Partikel (22, 22') zu einem ähnlich angeordneten seitlichen und unteren Teil aller gespeicherten Assay-Tröpfchen angezogen werden.

3. Mikrofluidischer tröpfchenbasierter Assay-Prozess nach einem vorhergehenden Anspruch, **dadurch gekennzeichnet, dass** das Magnetfeld in Bezug auf die gespeicherten Assay-Tröpfchen asymmetrisch ist.

4. Mikrofluidischer tröpfchenbasierter Assay-Prozess nach einem vorhergehenden Anspruch, **dadurch gekennzeichnet, dass** er das Nachweisen einer Fluoreszenz von fluoreszierenden nachweisbaren Markern und/oder das Nachweisen von Radioisotopmarkierten nachweisbaren Markern umfasst.

5. Mikrofluidischer tröpfchenbasierter Assay-Prozess nach einem vorhergehenden Anspruch, **dadurch gekennzeichnet, dass** er das Bereitstellen, in dem Assay-Tröpfchen, von zusätzlichen nachweisbaren Markern umfasst, die dazu in der Lage sind, an spezifische Proteine zu binden, die an der Oberfläche der biologischen Zelle (10) exprimiert sind, um die biologische Zelle (10) zu markieren.

6. Mikrofluidischer tröpfchenbasierter Assay-Prozess nach einem vorhergehenden Anspruch, **dadurch gekennzeichnet, dass** das flüssige Kulturmedium mindestens einen Vitalfarbstoff und/oder mindestens einen Nicht-Vitalfarbstoff umfasst.

7. Einrichtung für einen mikrofluidischen tröpfchenbasierten Assay-Prozess zum Analysieren der biologischen Zellaktivität durch einen Nachweis von mindestens einem Zielanalyten (16), der in einem oder mehreren Tröpfchen enthalten ist, wobei die Einrichtung eine Speicherkammer (42) zum Speichern von mehreren Assay-Tröpfchen (18) eines flüssigen Kulturmediums umfasst, die enthalten:
- paramagnetische Partikel (22, 22'),
- nachweisbare Marker (24),
- und mindestens eine biologische Zelle (10), die möglicherweise dazu in der Lage ist, mindestens einen Zielanalyten (16) freizusetzen,
wobei die Speicherkammer in einem Nachweisprozess der nachweisbaren Marker transparent ist und wobei die Speicherkammer zwischen zwei parallelen oberen und unteren inneren Wandflächen (47, 49) der Speicherkammer gebildet wird,
wobei sich die inneren Wandflächen parallel zu einer horizontalen Ebene erstrecken und vertikal durch eine Höhe getrennt sind, sodass die Assay-Tröpfchen entlang einer einzigen Schicht gespeichert sind,
wobei die Einrichtung einen Magnetfeldgenerator (70, 71) zum räumlichen Anordnen der paramagnetischen Partikel im Inneren der Assay-Tröpfchen beinhaltet, die in der Speicherkammer gespeichert sind,
und wobei die Einrichtung einen Detektor (60) einer physikalischen Eigenschaft der nachweisbaren Marker beinhaltet, die in einem gegebenen Assay-Tröpfchen enthalten sind, das in der Speicherkammer gespeichert ist,
**dadurch gekennzeichnet, dass** der Magnetfeldgenerator ein Magnetfeld generiert, sodass die paramagnetischen Partikel horizontal zu mindestens einer Seite der Speicherkammer und vertikal zu der unteren inneren Wandfläche der Speicherkammer angezogen werden.

8. Einrichtung nach Anspruch 7, **dadurch gekennzeichnet, dass** der Magnetfeldgenerator einen oder mehrere Magnete (71) umfasst, die eine Magnetbaugruppe (70) bilden, die unterhalb des Niveaus der unteren inneren Wandfläche (49) der Speicherkammer angeordnet ist.

9. Einrichtung nach Anspruch 7 oder 8, **dadurch gekennzeichnet, dass** die innere untere Wandfläche (49) der Speicherkammer zu einer unteren Wand (48) der Speicherkammer gehört, die eine untere äußere Wandfläche (51) gegenüber ihrer inneren Wandfläche aufweist, und dadurch, dass die Magnetbaugruppe unterhalb des Niveaus der unteren äußeren Wandfläche (51) angeordnet ist.

10. Einrichtung nach einem der Ansprüche 7 bis 9, **dadurch gekennzeichnet, dass** der Magnetfeldgenerator (70, 71) ein Magnetfeld generiert, sodass die paramagnetischen Partikel horizontal zu derselben Seite der Speicherkammer (42) angezogen werden.

11. Einrichtung nach einem der Ansprüche 7 bis 10, **dadurch gekennzeichnet, dass** der Magnetfeldgenerator einen oder mehrere Magnete (71) umfasst, die eine Magnetbaugruppe (70) bilden und jeweils Magnetpole aufweisen, wobei sich mindestens einer der Pole von jedem der Magnete horizontal zu derselben Seite der Speicherkammer (42) befindet.

12. Einrichtung nach einem der Ansprüche 7 bis 10, **dadurch gekennzeichnet, dass** der Magnetfeldgenerator einen oder mehrere Magnete (71) umfasst, die eine Magnetbaugruppe (70) bilden und jeweils Magnetpole aufweisen, wobei sich beide der Pole des einen oder der mehreren Magnete horizontal zu derselben Seite der Speicherkammer befinden.

13. Einrichtung nach einem der Ansprüche 7 bis 12, **dadurch gekennzeichnet, dass** die Speicherkammer (42) Teil einer Probenträgervorrichtung (44) ist, und dadurch, dass der Magnetfeldgenerator (70, 71) an der Probenträgervorrichtung befestigt ist.

14. Einrichtung nach einem der Ansprüche 7 bis 12, **dadurch gekennzeichnet, dass** der Magnetfeldgenerator (70, 71) Teil einer Nachweisstation (62) der Einrichtung ist, und dadurch, dass die Speicherkammer (42) Teil einer Probenträgervorrichtung (44) ist, wobei die Nachweisstation und die Probenträgervorrichtung in Bezug zueinander beweglich sind und zum Nachweis von mindestens einem Zielanalyten, der in einem oder mehreren Tröpfchen enthalten ist, die in der Speicherkammer der Probenträgervorrichtung enthalten sind, in Übereinstimmung gebracht werden.

15. Einrichtung nach einem der Ansprüche 7 bis 14, **dadurch gekennzeichnet, dass** die Probenträgervorrichtung (44) zwei Platten (46, 48) umfasst, welche die zwei Wände der Speicherkammer (42) bilden, und dadurch, dass die zwei Platten aneinander durch einen Klebefilm (50) befestigt sind, der zwischen den zwei Platten eingeschlossen ist, wobei die Dicke des Klebefilms vertikal die Höhe der Speicherkammer bestimmt.

16. Einrichtung nach Anspruch 15, **dadurch gekennzeichnet, dass** der Klebefilm (50) einen inneren Ausschnitt (52) umfasst, dessen Grenze (54) die Erstreckung der Speicherkammer (42) entlang der horizontalen Ebene begrenzt.

## Revendications

1. Procédé de test microfluidique basé sur des gouttelettes pour analyser l'activité biologique d'une cellule par détection d'au moins un analyte cible (16) contenu dans une ou plusieurs gouttelettes, lequel procédé comprend l'étape de création de plusieurs gouttelettes de test (18) d'un milieu de culture liquide, où une gouttelette de test contient :
- des particules paramagnétiques (22, 22') ;
- des marqueurs détectables (24) ; et
- au moins une cellule biologique (10) potentiellement capable de libérer au moins un analyte cible (16) ;
dans lequel les particules paramagnétiques (22, 22') et les marqueurs détectables (24) sont capables de se lier à un analyte cible (16) pour former une combinaison marquée et magnétisée (25) avec un analyte cible (16),
lequel procédé comprend le stockage de plusieurs gouttelettes de test le long d'une seule couche le long d'un plan de stockage horizontal,
lequel procédé comprend la génération d'un champ magnétique à travers les gouttelettes de test stockées pour arranger spatialement les particules paramagnétiques à l'intérieur des gouttelettes de test stockées,
et lequel procédé comprend la détection, dans une gouttelette de test stockée donnée, d'une caractéristique physique des marqueurs détectables contenus dans la gouttelette de test donnée, par imagerie optique des marqueurs détectables,
**caractérisé en ce que** la génération d'un champ magnétique génère un champ magnétique de façon que les particules paramagnétiques sont attirées vers une partie latérale et inférieure de la gouttelette de test stockée donnée dans laquelle elles sont contenues.

2. Procédé de test microfluidique basé sur des gouttelettes selon la revendication 1, **caractérisé en ce que** le champ magnétique est tel que les particules paramagnétiques (22, 22') sont attirées vers une partie latérale et inférieure disposée similairement de toutes les gouttelettes de test stockées.

3. Procédé de test microfluidique basé sur des gouttelettes selon l'une quelconque des revendications précédentes, **caractérisé en ce que** le champ magnétique est asymétrique par rapport aux gouttelettes de test stockées.

4. Procédé de test microfluidique basé sur des gouttelettes selon l'une quelconque des revendications précédentes, **caractérisé en ce qu'**il comprend la détection de la fluorescence de marqueurs détectables fluorescents et/ou la détection de marqueurs détectables marqués par un isotope radioactif.

5. Procédé de test microfluidique basé sur des gouttelettes selon l'une quelconque des revendications précédentes, **caractérisé en ce qu'**il comprend la disposition, dans la gouttelette de test, de marqueurs détectables additionnels capables de se lier à des protéines spécifiques exprimées sur la surface de la cellule biologique (10) pour étiqueter la cellule biologique (10).

6. Procédé de test microfluidique basé sur des gouttelettes selon l'une quelconque des revendications précédentes, **caractérisé en ce que** le milieu de culture liquide comprend au moins un colorant vital et/ou au moins un colorant non vital.

7. Dispositif pour un procédé de test microfluidique basé sur des gouttelettes pour analyser l'activité biologique d'une cellule par détection d'au moins un analyte cible (16) contenu dans une ou plusieurs gouttelettes, lequel dispositif comprend une chambre de stockage (42) pour stocker plusieurs gouttelettes de test (18) d'un milieu de culture liquide contenant :
- des particules paramagnétiques (22, 22') ;
- des marqueurs détectables (24) ; et
- au moins une cellule biologique (10) potentiellement capable de libérer au moins un analyte cible (16) ;
où la chambre de stockage est transparente dans un procédé de détection des marqueurs détectables et où la chambre de stockage est formée entre deux surfaces internes de parois parallèles supérieure et inférieure (47, 49) de la chambre de stockage, les surfaces internes de parois s'étendant parallèlement à un plan horizontal et étant séparées verticalement par une hauteur telle que les gouttelettes de test soient stockées le long d'une seule couche ;
lequel dispositif comprend un générateur de champ magnétique (70, 71) pour arranger spatialement les particules paramagnétiques à l'intérieur des gouttelettes de test stockées dans la chambre de stockage,
et lequel dispositif comprend un détecteur (60) d'une caractéristique physique des marqueurs détectables contenus dans une gouttelette de test donnée dans la chambre de stockage,
**caractérisé en ce que** le générateur de champ magnétique génère un champ magnétique de façon que les particules paramagnétiques sont attirées horizontalement en direction d'au moins un côté de la chambre de stockage et verticalement en direction de la surface interne de paroi inférieure de la chambre de stockage.

8. Dispositif selon la revendication 7, **caractérisé en ce que** le générateur de champ magnétique comprend un ou plusieurs aimants (71) formant un assemblage d'aimants (70) qui est disposé sous le niveau de la surface interne de paroi inférieure (49) de la chambre de stockage.

9. Dispositif selon la revendication 7 ou 8, **caractérisé en ce que** la surface interne de paroi inférieure (49) appartient à une paroi inférieure (48) de la chambre de stockage qui présente une surface externe de paroi inférieure (51) opposée à sa surface interne de paroi, et **en ce que** l'assemblage d'aimants est disposé sous le niveau de la surface externe de paroi inférieure (51).

10. Dispositif selon l'une quelconque des revendications 7 à 9, **caractérisé en ce que** le générateur de champ magnétique (70, 71) génère un champ magnétique de façon que les particules paramagnétiques sont attirées horizontalement en direction d'un même côté de la chambre de stockage (42).

11. Dispositif selon l'une quelconque des revendications 7 à 10, **caractérisé en ce que** le générateur de champ magnétique comprend un ou plusieurs aimants (71) formant un assemblage d'aimants (70) et ayant chacun des pôles magnétiques, dans lequel au moins un des pôles de chacun des aimants est situé horizontalement à d'un même côté de la chambre de stockage (42).

12. Dispositif selon l'une quelconque des revendications 7 à 10, **caractérisé en ce que** le générateur de champ magnétique comprend un ou plusieurs aimants (71) formant un assemblage d'aimants (70) et ayant chacun des pôles magnétiques, dans lequel les deux pôles du ou des aimants sont situés horizontalement d'un même côté de la chambre de stockage.

13. Dispositif selon l'une quelconque des revendications 7 à 12, **caractérisé en ce que** la chambre de stockage (42) fait partie d'un dispositif porte-échantillons (44) et **en ce que** le générateur de champ magnétique (70, 71) est fixé audit dispositif porte-échantillons.

14. Dispositif selon l'une quelconque des revendications 7 à 12, **caractérisé en ce que** le générateur de champ magnétique (70, 71) fait partie d'un poste de détection (62) du dispositif et **en ce que** la chambre de stockage (42) fait partie d'un dispositif porte-échantillons (44), le poste de détection et le dispositif porte-échantillons étant mobiles l'un par rapport à l'autre et étant mis en coïncidence pour la détection d'au moins un analyte cible contenu dans une ou plusieurs gouttelettes contenues dans la chambre de stockage du dispositif porte-échantillons.

15. Dispositif selon l'une quelconque des revendications 7 à 14, **caractérisé en ce que** le dispositif porte-échantillons (44) comprend deux plaques (46, 48) formant les deux parois de la chambre de stockage (42), et **en ce que** les deux plaques sont fixées l'une à l'autre par un film adhésif (50) pris en sandwich entre les deux plaques, dans lequel l'épaisseur du film adhésif détermine verticalement la hauteur de la chambre de stockage.

16. Dispositif selon la revendication 15, **caractérisé en ce que** le film adhésif (50) comprend une découpe interne (52), dont la bordure (54) délimite l'extension de la chambre de stockage (42) le long du plan horizontal.
